# EUROPÄISCHE PATENTSCHRIFT

(11) **EP 3 153 121 B1**
(45) Veröffentlichungstag und Bekanntmachung des Hinweises auf die Patenterteilung: **07.03.2018**
(21) Anmeldenummer: 16189604.8
(22) Anmeldetag: 20.09.2016
(51) Int. Cl.: A61B 17/88, B01F 11/00, B01F 13/00, B01F 15/02, A61B 90/00, B67B 7/92

(54) **VORRICHTUNG UND VERFAHREN ZUM ÖFFNEN VON GLASAMPULLEN SOWIE ZEMENTIERVORRICHTUNG**
A DEVICE AND METHOD FOR MANUALLY OPENING GLASS AMPULES AND A CEMENTING DEVICE
DISPOSITIF ET PROCÉDÉ D'OUVERTURE D'AMPOULES EN VERRE ET DISPOSITIF DESTINÉ À CIMENTER

(30) Priorität: 25.09.2015 DE 102015116245
(43) Veröffentlichungstag der Anmeldung: 12.04.2017
(73) Patentinhaber: Heraeus Medical GmbH, 61273 Wehrheim (DE)
(72) Erfinder: Vogt, Sebastian Dr., 99092 Erfurt (DE)
(74) Vertreter: Schultheiss & Sterzel Patentanwälte PartG mbB

(56) Entgegenhaltungen:
- WO-A1-2011/003625
- DE-A1-102010 026 496
- US-A1- 2006 049 203
- US-A1- 2012 160 867

## Beschreibung

Die Erfindung betrifft eine Vorrichtung zum manuellen Öffnen von Glasampullen innerhalb der Vorrichtung sowie eine medizinische Zementiervorrichtung mit einer solchen Vorrichtung. Des Weiteren betrifft die Erfindung ein Verfahren zum Öffnen von Glasampullen in einer solchen Vorrichtung.

Gegenstand der Erfindung sind somit eine Vorrichtung zum Öffnen von Glasampullen, ein Verfahren zur Öffnung von Glasampullen und medizinische Zementiervorrichtungen, insbesondere Full-Prepack-Mischsysteme, mit der Vorrichtung zum Öffnen von Glasampullen für die Lagerung und Anmischung von Komponenten von PMMA-Knochenzement.

Kleine Volumina von leichtflüchtigen organischen Flüssigkeiten, wie zum Beispiel Methylmethacrylat, können in Plastikbeuteln, Plastikbehältern und in Glasampullen gelagert werden. Glasampullen haben gegenüber Packmitteln aus Kunststoff beziehungsweise aus Kunststoffmehrschichtsystemen den entscheidenden Vorteil, dass sie komplett undurchlässig sind. Es tritt keine Migration von Flüssigkeiten durch die Glaswand auf. Bei Packmitteln aus Kunststoff und Kunststoffmehrschichtsystemen werden immer geringe Verluste infolge der Migration der organischen Flüssigkeit durch die Kunststoffschichten beobachtet. Weiterhin ist die Herstellung und Befüllung von Glasampullen kostengünstig großtechnisch möglich.

Für Polymethylmethacrylat-Knochenzemente (PMMA-Knochenzemente), die aus einer flüssigen Monomerkomponente und einer Pulverkomponente aufgebaut sind, wird die Monomerflüssigkeit üblicherweise in Glasampullen aufbewahrt (Kühn, K.-D.: Knochenzemente für die Endoprothetik, Springer Verlag Berlin Heidelberg New York, 2001). Glasampullen können durch Brechen der Glaswand an einer vorgegebenen Sollbruchstelle geöffnet werden. Jedoch besteht bei der direkten manuellen Öffnung der Glasampullen die Gefahr, dass der Anwender Schnittverletzungen erleidet.

Eine vorteilhafte Entwicklung in der Zementiertechnik stellen Zementiervorrichtungen dar, in denen sowohl das Zementpulver als auch die Monomerflüssigkeit bereits in separaten Kompartimenten der Mischsysteme verpackt sind und die erst unmittelbar vor der Zementapplikation in der Zementiervorrichtung miteinander vermischt werden.

Solche Full-Prepacked-Zementiervorrichtungen wurden mit der EP 0 692 229 A1, der DE 10 2009 031 178 B3, der US 5,997,544 A, der US 6,709,149 B1, der DE 698 12 726 T2 und der US 5,588,745 A vorgeschlagen.

Es wurde eine Reihe von Vorrichtungen beschrieben, mit denen Glasampullen innerhalb der Vorrichtungen geöffnet werden können. Bei diesen Vorrichtungen wird die Gefahr von Schnittverletzungen des Anwenders sicher ausgeschlossen. Weiterhin wird der Anwender bei Lagerung von gesundheitsgefährdenden Flüssigkeiten in den Glasampullen vor direktem Kontakt mit den Flüssigkeiten während des Öffnens und des Leerens der Glasampullen durch die Vorrichtungen geschützt.

Man kann Vorrichtungen zur Öffnung von Glasampullen in zwei Gruppen einteilen. Bei einer ersten Gruppe wird der Kopf der Ampulle gegenüber dem Ampullenkörper durch Scherung abgetrennt. Bei der zweiten Gruppe wird der Ampullenkörper beziehungsweise der Ampullenboden selbst gebrochen.

Aus der DE 198 41 722 A1 ist eine geschlossene Vorrichtung bekannt, bei der der Ampullenkopf in einer drehbaren Welle gelagert ist und der Ampullenkörper in einem geschlossenen Behälter fixiert ist. Bei Drehung der Welle von außen wird der Ampullenkopf vom Ampullenkörper durch Scherung abgetrennt.

Eine ähnliche Vorrichtung ist in der DE 29 21 565 A1 offenbart. Dabei ist ein hohler Hebel auf einem Ampullenkopf aufgesteckt, wobei die Ampulle in einem Behälter fixiert ist. Von außen kann der Hebel bewegt werden, wodurch der Ampullenkopf von dem Ampullenkörper abgebrochen wird.

Die US 2006/0049203 A1 offenbart einen Applikator für einen polymerisierbaren Klebstoff auf Basis eines Monomers, bei dem eine Brechvorrichtung mit einem beweglichen Betätigungselement bedient werden kann, um eine Ampulle im Inneren des Applikators aufzubrechen.

In der EP 0 079 983 A1 wird eine Zementiervorrichtung vorgeschlagen, bei welcher der Ampullenkopf in einem starren walzenförmigen Körper gelagert ist, der an einer Kartusche befestigt ist, wobei ein Ampullenhalter, der den Ampullenkörper aufnimmt, drehbar um die Längsachse des walzenförmigen Körpers angebracht ist. Wenn der Ampullenhalter um die Drehachse des starren walzenförmigen Körpers gedreht wird, dann bricht der Ampullenkörper vom sich nicht mit drehenden Ampullenkopf ab und die Flüssigkeit kann dann in die anliegende Kartusche durch Vakuumeinwirkung gesaugt werden.

Eine nach dem gleichen Prinzip wirkende Vorrichtung wird mit der WO 2010/012 114 A1 vorgeschlagen. Dort ist der Ampullenkopf in einem von außen manuell zu drehenden Walzenkörper angeordnet, wobei der Walzenkörper in einer zylinderförmigen Öffnung eines starren Ampullenhalters angeordnet ist. Wenn der Walzenkörper manuell gedreht wird, dann bricht bei der Drehbewegung des Walzenkörpers der mitdrehende Ampullenkopf vom fixierten Ampullenkörper ab und die Ampulle wird dadurch geöffnet. Nachteilig an dieser Vorrichtung ist die relativ aufwendige Abdichtung des Walzenkörpers in der zylinderförmigen Öffnung des Ampullenhalters. Ein selbstständiges Auslaufen von Flüssigkeiten aus der gebrochenen Ampulle ist nur möglich, wenn die Ampulle einen Winkel von ca. 45° zur Senkrechten hat oder wenn bei senkrechter Lagerung des Ampullenkörpers der Querschnitt des Ampullenhalses so groß ist, dass die Oberflächenspannung der Flüssigkeit durch die Schwerkraft überkompensiert wird.

In der DE 10 2010 026 496 A1 wird eine einfache, robuste Vorrichtung zur Öffnung von Glasampullen beschrieben. Bei dieser Vorrichtung ist der Ampullenkörper in einem elastisch verformbaren Ampullenhalter angeordnet, der mit einem starren Hohlkörper zur Aufnahme eines Ampullenkopfs verbunden ist. Der Ampullenkopf liegt dabei in einem starren Hohlzylinder, der einen Durchmesser größer oder gleich der Länge des Ampullenkopfs hat. Bei der Bewegung des Ampullenhalters entgegengesetzt zur Längsachse des Ampullenhalters wird der Ampullenkörper vom Ampullenkopf abgebrochen. Der Ampullenkopf fällt nach unten und kann sich im Hohlzylinder auf die Seite drehen und bleibt auf einerflüssigkeitsdurchlässigen Porenscheibe liegen, durch welche die Flüssigkeit aus der Ampulle fließen kann.

In der EP 0 972 499 A2 ist beschrieben, dass eine Ampulle in einem zweiteiligen Ampullenhalter gelagert wird. Beide Teile des Ampullenhalters enthalten Gewindegänge und können dadurch miteinander verschraubt werden. Bei der Verschraubung der beiden Teile des Ampullenhalters wird die in dem Ampullenhalter angeordnete Ampulle mit ihrem Ampullenboden gegen einen kegelförmigen Keil gepresst. Dadurch bricht der Ampullenboden und die Flüssigkeit kann aus der Ampulle auslaufen.

Die Aufgabe der Erfindung besteht darin, die Nachteile des Stands der Technik zu überwinden. Insbesondere soll eine einfach zu handhabende Vorrichtung zur Öffnung von Glasampullen bereitgestellt werden, die möglichst platzsparend ist und eine sichere Öffnung der Glasampullen erlaubt, so dass die komplette Flüssigkeit unabhängig von ihrer Oberflächenspannung unmittelbar aus den Ampullen ausläuft. Eine weitere Aufgabe besteht darin, ein Verfahren zur Öffnung von Glasampullen bereitzustellen. Die zu entwickelnde Vorrichtung zur Öffnung von Glasampullen soll in Full-Prepac-Zementiervorrichtungen für Pulver-Flüssigkeits-Polymethylmethacrylat-Knochenzemente integriert werden. Die Vorrichtung soll kostengünstig in der Herstellung sein und eine fehlerfreie Anwendung ermöglichen. Des Weiteren soll die Vorrichtung ohne Energiespeicher auskommen. Für medizinische Anwendungen soll die Vorrichtung als Wegwerfprodukt ausgelegt sein, so dass möglichst keine Metalle verwendet werden sollten und ein möglichst weitgehender Aufbau aus Kunststoff angestrebt wird. Als medizinisches Mischsystem soll die Vorrichtung zudem die Lagerung und Vermischung einer Monomer-Flüssigkeit in einer Ampulle mit einem Knochenzementpulver in einer Kartusche ermöglichen.

Des Weiteren soll eine kostengünstig zu fertigende und zuverlässig funktionierende Zementiervorrichtung zum Mischen eines medizinischen Zements und gegebenenfalls zur Lagerung der Ausgangskomponenten sowie ein Verfahren zum Öffnen der Glasampulle und zum Mischen des Knochenzements gefunden werden, bei dem eine möglichst einfache manuelle Bedienung angewendet werden kann.

Die Hauptkomponente des Polymethylmethacrylat-Knochenzements als Mischgut soll ein Pulver sein und die zweite Komponente soll in Form einer Flüssigkeit in der Glasampulle vorliegen. Die beiden Ausgangskomponenten des Knochenzements sollen bevorzugt in dem Vakuummischsystem getrennt gelagert werden können und durch Anwendung der Vorrichtung sicher zusammengeführt werden können.

Die Aufgaben der Erfindung werden gelöst durch eine Vorrichtung zum manuellen Öffnen von Glasampullen innerhalb der Vorrichtung, wobei die Vorrichtung die folgenden Teile aufweist:
A) eine Aufnahme mit zumindest bereichsweise geschlossenen Seitenwänden zur Aufnahme der Glasampulle, wobei die Aufnahme zumindest eine verformbare geschlossene Seitenwand aufweist und gegenüber der verformbaren Seitenwand ein Stützelement vorgesehen ist,
B) ein Sieb und/oder ein Filter das, der oder die unterhalb der Aufnahme angeordnet ist oder sind, so dass der Inhalt der geöffneten Glasampulle durch das Sieb und/oder den Filter fließt,
C) ein erster Hebel, der um eine erste Achse drehbar gegen die Aufnahme gelagert ist, wobei ein freies Ende des ersten Hebels gegen die verformbare Seitenwand der Aufnahme drückbar ist,
D) ein zweiter Hebel, der um eine zweite Achse drehbar gegen die Aufnahme gelagert ist, wobei die zweite Achse den zweiten Hebel in einen kurzen Hebelarm und einen langen Hebelarm teilt, wobei ein Ende des kurzen Hebelarms durch manuelles Bedienen des langen Hebelarms gegen den ersten Hebel zu drücken ist, so dass das freie Ende des ersten Hebels gegen die verformbare Seitenwand drückt und diese derart verformt, dass eine in der Aufnahme befindliche und zur Aufnahme passende Glasampulle durch den Druck des freien Endes des ersten Hebels aufzubrechen ist.

Eine Glasampulle kann im Sinne der vorliegenden Erfindung als zur Aufnahme passend angesehen werden, wenn die seitlichen Wandungen der Glasampulle an zumindest zwei gegenüberliegenden Seiten an der Aufnahme anliegen, wenn die Glasampulle in die Aufnahme eingesteckt ist. Bevorzugt liegen die Wandungen einer in die Aufnahme eingesteckten Glasampulle an den Seitenwänden der Aufnahme an.

Die Richtungsbezeichnungen oben und unten beziehen sich auf die Aufstellung der Vorrichtung beziehungsweise auf die Flussrichtung der Flüssigkeit aus der Glasampulle nach Öffnen derselben innerhalb der Vorrichtung. Die Flüssigkeit aus der geöffneten Glasampulle fließt also nach unten aus der Glasampulle aus durch das Sieb und/oder den Filter.

Es kann erfindungsgemäß vorgesehen sein, dass die verformbare Seitenwand elastisch und/oder plastisch verformbar ist. Die verformbare Seitenwand soll aber im Rahmen der zum Öffnen vorgesehen Verformung zerstörungsfrei verformbar sein.

Es kann vorgesehen sein, dass unterhalb der Aufnahme ein Trichter vorgesehen ist, der zur Aufnahme und Weiterleitung einer Flüssigkeit aus der geöffneten Glasampulle vorgesehen ist.

Bevorzugt weisen die Glasampullen einen zylindrischen Ampullenkörper auf.

Das Stützelement muss lediglich dazu ausreichen, die Glasampulle in Position zu halten, bis die Glasampulle aufgrund einer Krafteinwirkung durch die verformbare Seitenwand aufbricht.

Bei erfindungsgemäßen Vorrichtung kann vorgesehen sein, dass die Aufnahme ein Hohlzylinder ist und/oder die Aufnahme aus einem Elastomer besteht oder einen Einsatz aus einem Elastomer umfasst, wobei vorzugsweise das Elastomer eine Shore-Härte größer 60 aufweist, wobei besonders bevorzugt das Elastomer ein Silikonkautschuk oder ein Ethylen-Propylen-Dien-Kautschuk (EPDM) ist.

Durch die zylindrische Geometrie der Aufnahme wird erreicht, dass eine zylindrische Glasampulle mit festem Sitz in die Aufnahme eingesteckt werden kann. Mit der angegebenen Härte nach Shore wird erreicht, dass die Glasampulle sicher innerhalb der Aufnahme geöffnet werden kann, ohne dass die Aufnahme zerstört wird oder ein Leck entwickelt.

Gemäß einer bevorzugten Weiterbildung der vorliegenden Erfindung kann vorgesehen sein, dass in der Aufnahme ein Absatz zur Auflage der Glasampulle angeordnet ist, wobei der Absatz kleiner als die Hälfte der Fläche des Ampullenbodens oder des Ampullenquerschnitts ist.

Hierdurch wird die Glasampulle in der Aufnahme fixiert, so dass sie in definierter Position mit Hilfe der Hebel aufzubrechen ist und nicht ausweichen kann. Der Absatz ist vorzugsweise so in der Aufnahme angeordnet, dass der Abstand zwischen dem Absatz und dem flüssigkeitsdurchlässigen Sieb und/oder Filter gleich oder größer ist als der Durchmesser der Glasampulle.

Bei Ausführungsformen mit Absatz kann vorgesehen sein, dass der Absatz derart in der Aufnahme angeordnet ist, dass der Abstand zwischen dem Absatz und dem Sieb und/oder Filter gleich oder größer ist als der Außendurchmesser der einzusetzenden Glasampulle.

Hiermit kann sichergestellt werden, dass der Ampullenboden vollständig in dem Zwischenraum zwischen dem Absatz und dem Sieb und/oder Filter aufgenommen werden kann, ohne den Flüssigkeitsstrom aus der geöffneten Glasampulle zu blockieren.

Ferner kann bei Ausführungsformen mit Absatz vorgesehen sein, dass der Absatz derart in der Aufnahme angeordnet ist, dass die Glasampulle so auf dem Absatz steht, dass das freie Ende des ersten Hebels oberhalb des Ampullenbodens auf der Außenseite der verformbaren Seitenwand liegt.

Damit wird erreicht, dass die Kraft des ersten Hebels im Bereich des Ampullenbodens wirkt und so der Ampullenboden abgebrochen werden kann.

Des Weiteren wird mit der vorliegenden Erfindung vorgeschlagen, dass das freie Ende des ersten Hebels bei einer Bedienung des zweiten Hebels derart auf die verformbare Seitenwand drückt, dass der Vektor der Kraft eine Komponente aufweist, die in Richtung des Siebs und/oder Filters gerichtet ist und/oder die eine in die Aufnahme eingesetzte Glasampulle in die Aufnahme hinein drückt, vorzugsweise in Richtung des Absatzes drückt.

Hierdurch wird erreicht, dass die Glasampulle beim Bedienen der Hebel nicht aus der Aufnahme herausgedrückt werden kann und die Positionierung der Glasampulle sichergestellt ist. Dies führt dazu, dass die Glasampulle sicher aufgebrochen wird und nicht durch eine Bewegung aus der Aufnahme hinaus ausweichen kann.

Bei einer bevorzugten Ausführung kann vorgesehen sein, dass am freien Ende des ersten Hebels auf der zur Aufnahme zugewandten Seite eine Schneidkante angeordnet ist.

Mit der Schneidkante wird eine definierte Kraft auf eine kleine Fläche der Glasampulle ausgeübt, so dass die Glasampulle mit möglichst geringem Kraftaufwand an einer möglichst genau definierten Stelle aufzubrechen ist. Diese Schneidkante kann dreieckig oder keilförmig ausgebildet sein. Es ist auch möglich, dass die Schneidkante pyramidenförmig, prismenförmig oder kegelförmig ausgebildet ist.

Zur besseren Kraftübertragung kann erfindungsgemäß vorgesehen sein, dass das Längenverhältnis des langen Hebelarms zum kurzen Hebelarm mindestens 5 zu 1 ist.

Hiermit wird eine Kraftübersetzung erreicht, so dass ein manuelles Öffnen der Glasampulle in der Vorrichtung ohne großen Kraftaufwand manuell möglich ist.

Ferner kann vorgesehen sein, dass in der Aufnahme eine Glasampulle angeordnet ist, die eine Flüssigkeit enthält, vorzugsweise eine Monomerflüssigkeit zur Herstellung eines medizinischen Knochenzements enthält.

Hierdurch kann die Vorrichtung unmittelbar zum Öffnen der Glasampulle eingesetzt werden, ohne dass vorher die Glasampulle eingesetzt werden müsste.

Gemäß einer bevorzugten Weiterbildung der vorliegenden Erfindung kann vorgesehen sein, dass der zweite Hebel in der gleichen Ebene zu drehen ist, wie der erste Hebel, wobei die Bewegung des zweiten Hebels in die Bewegung des ersten Hebels eingreift.

Hiermit wird sichergestellt, dass der erste Hebel mit dem zweiten Hebel bewegt beziehungsweise bedient werden kann.

Des Weiteren kann vorgesehen sein, dass die zweite Achse des zweiten Hebels oberhalb der ersten Achse des ersten Hebels angeordnet ist, wobei vorzugsweise die erste Achse des ersten Hebels und die zweite Achse des zweiten Hebels parallel zueinander angeordnet sind.

Damit wird erreicht, dass der erste Hebel mit dem zweiten Hebel von oben bedient werden kann. Besonders bevorzugt kann vorgesehen sein, dass der lange Hebelarm des zweiten Hebels oberhalb der zweiten Achse angeordnet und der kurze Hebelarm unterhalb der zweiten Achse angeordnet. Das freie Ende des ersten Hebels ist dazu oberhalb der ersten Achse angeordnet. Ferner kann vorgesehen sein, dass der kurze Hebelarm des zweiten Hebels an dem freien Ende des ersten Hebels anliegt. Des Weiteren kann vorgesehen sein, dass der kurze Hebelarm des zweiten Hebels zwischen der zweiten Achse des zweiten Hebels und dem freien Ende oder der ersten Achse des ersten Hebels angeordnet ist. Mit all diesen Maßnahmen wird die Bedienbarkeit der Vorrichtung verbessert.

Die der vorliegenden Erfindung zugrundeliegenden Aufgaben werden auch gelöst durch eine medizinische Zementiervorrichtung, insbesondere ein Full-Prepacked-Zementiervorrichtung zum Mischen eines PMMA-Knochenzements, aufweisend eine erfindungsgemäße Vorrichtung zum manuellen Öffnen von Glasampullen innerhalb der Vorrichtung, ferner aufweisend eine Kartusche mit einem Mischraum enthaltend ein Zementpulver, insbesondere ein Knochenzementpulver, wobei der Mischraum mit der Aufnahme unterhalb des Siebs oder des Filters flüssigkeitsdurchlässig verbunden ist.

Die Vorrichtung lässt sich im Zusammenhang mit medizinischen Zementiervorrichtungen besonders nutzbringend einsetzen.

Dabei kann vorgesehen sein, dass in der Aufnahme eine Glasampulle angeordnet ist, in der eine Monomerflüssigkeit enthalten ist, eine Pumpe, mit der die Monomerflüssigkeit in die Kartusche zu pumpen ist, eine Verbindungsleitung, mit dem die Monomerflüssigkeit aus der Glasampulle in die Kartusche zu leiten ist, eine manuell zu bedienende Rühreinheit, mit der der Inhalt der Kartusche durchmischbar ist, einen Förderkolben, der in Längsrichtung verschiebbar in der Kartusche vorgesehen ist und mit der gemischt Knochenzement aus der Kartusche auszutreiben ist, einem gasdurchlässigen Verbindungsmittel im Förderkolben, das den Innenraum der Kartusche mit der äußeren Umgebung verbindet und ein Fußteil, das mit der Aufnahme, der Kartusche und den Hebeln verbunden ist und mit dem die Zementiervorrichtung auf eine ebene Fläche aufstellbar ist.

Die Pumpe kann manuell oder mit einer vorgespannten Feder angetrieben werden.

Hiermit wird eine fertige medizinische Zementiervorrichtung bereitgestellt, die die Vorrichtung zum Öffnen der Glasampulle nutzt.

Mit der Erfindung wird auch vorgeschlagen, dass der Förderkolben den Mischraum der Kartusche bis auf die gasdurchlässige Verbindung gasdicht verschließt.

Bei solchen Zementiervorrichtungen kann auch vorgesehen sein, dass die Pumpe eine Saugdruckpumpe ist wobei der Druck-Pumpenteil der Saugdruckpumpe mit der Aufnahme verbunden ist und der Saug-Pumpenteil der Saugdruckpumpe mit dem Mischraum der Kartusche verbunden ist, vorzugsweise über das gasdurchlässige Verbindungsmittel im Förderkolben mit dem Mischraum der Kartusche verbunden ist.

Die der vorliegenden Erfindung zugrundeliegenden Aufgaben werden ferner gelöst durch ein Verfahren zum Öffnen einer Glasampulle in einer solchen Vorrichtung oder Zementiervorrichtung bei der der lange Hebelarm des zweiten Hebels manuell um die zweite Achse gedreht wird, wodurch der kurze Hebelarm des zweiten Hebels auf das freie Ende des ersten Hebels gedrückt wird, so dass sich der erste Hebel um die erste Achse dreht, und das freie Ende des ersten Hebels auf die verformbare Seitenwand der Aufnahme drückt, die verformbare Seitenwand verformt und durch die Verformung der verformbaren Seitenwand eine Glasampulle in der Aufnahme mechanisch aufgebrochen wird.

Dabei kann vorgesehen sein, dass die Glasampulle vor dem manuellen Bedienen des zweiten Hebels in die Aufnahme eingesteckt wird, bevorzugt mit dem Ampullenboden der Glasampulle voraus in die Aufnahme eingesteckt wird.

Des Weiteren kann vorgesehen sein, dass nach dem Aufbrechen der Glasampulle eine Monomer-Flüssigkeit aus der Glasampulle durch das Sieb und/oder den Filter ausläuft und in einen Mischraum der Kartusche geleitet wird, in dem sich ein Knochenzementpulver befindet, wobei anschließend das Knochenzementpulver und die Monomerflüssigkeit in dem Mischraum der Kartusche gemischt werden.

Der Erfindung liegt die überraschende Erkenntnis zugrunde, dass es mit der erfindungsgemäßen Vorrichtung gelingt, eine Glasampulle großflächig innerhalb der Vorrichtung beziehungsweise der Zementiervorrichtung aufzubrechen, so dass die Monomer-Flüssigkeit aus der Glasampulle in kurzer Zeit ausströmt und zum Mischen mit einem medizinischen Knochenzementpulver bereitsteht. Mit Hilfe der beiden Hebel, die in Wechselwirkung miteinander stehen, ist es möglich, den Druck auf die Glasampulle in Richtung des Sitzes der Glasampulle in der Aufnahme zu richten, so dass ein Ausweichen der Glasampulle aus der Aufnahme heraus ausgeschlossen werden kann. Gleichzeitig kann ein sehr genau definierter lokaler Druck auf die Glasampulle ausgeübt werden, mit dem die Glasampulle in der Vorrichtung aufgebrochen werden kann. Mit Hilfe der verformbaren Seitenwand kann dafür gesorgt werden, dass die Kraft durch diese Seitenwand ins Innere der Aufnahme auf die Glasampulle übertragen wird, wobei die Aufnahme dabei geschlossen bleibt. Ein Austreten der Flüssigkeit aus der Aufnahme kann somit ausgeschlossen werden. Mit Hilfe des Siebs und/oder des Filters können Glassplitter, die beim Öffnen der Glasampulle entstehen können, zurückgehalten werden. Die Monomerflüssigkeit ist dann zum Mischen mit dem Knochenzementpulver nutzbar.

Der besondere Vorteil der erfindungsgemäßen Vorrichtung besteht auch darin, dass beliebige Ampullen, unabhängig von der Ampullenlänge und der Geometrie des Ampullenkopfs, sicher geöffnet werden können, wenn der Ampullendurchmesser gleich oder etwas größer ist als der Innendurchmesser des Ampullenhalters beziehungsweise der Aufnahme. Weiterhin ist es ein besonderer Vorteil, dass beim Brechen der Ampullenwand die in der Ampulle enthaltene Flüssigkeit unabhängig von der Oberflächenspannung sofort komplett ausfließt. Dagegen fließt bei konventionellen Ampullenbrechern die Flüssigkeit nach Abtrennen des Ampullenkopfs durch den relativ engen Querschnitt des Ampullenhalses deutlich langsamer aus. Dabei werden halbwegs große Auslaufgeschwindigkeiten nur erzielt, wenn entweder die Ampulle im Winkel von ca. 45° mit dem Ampullenhals nach unten gehalten wird oder wenn der Querschnitt des Ampullenhalses so groß ist, dass die Oberflächenspannung der Flüssigkeit den Meniskus der Flüssigkeit nicht im Ampullenhals halten kann.

Eine beispielhafte erfindungsgemäße Vorrichtung zum Öffnen von Glasampullen, kann beispielsweise zusammengesetzt sein aus
a) einem Hohlzylinder als Aufnahme mit mindestens einer verformbaren Fläche zur Aufnahme von mindestens einer Glasampulle,
b) einem nicht verformbaren Stützelement gegenüber der verformbaren Mantelfläche des Hohlzylinders,
c) einer flüssigkeitsdurchlässigen Siebplatte, die unter unterhalb des Hohlzylinders angeordnet ist,
d) einem Trichter der unterhalb der Siebplatte angeordnet ist,
e) einem ersten Hebel, der an einem ersten Gelenk derart drehbar angelenkt ist, dass das freie Ende des Hebels auf der elastisch verformbaren Außenfläche des Hohlzylinders anliegt,
f) einem zweiten Hebel, der durch ein zweites Gelenk in einen kurzen Hebelabschnitt und einem langen Hebelabschnitt geteilt ist, wobei der Drehpunkt des zweiten Hebels gleich oder höher angeordnet ist als der Drehpunkt des ersten Hebels,
g) der zweite Hebel parallel zum Hohlzylinder angeordnet ist, wobei der kurze Hebelabschnitt parallel zum ersten Hebel angeordnet ist und
h) wobei bei Drehung des langen Hebelabschnitts des zweiten Hebels um den zweiten Drehpunkt entgegengesetzt zum Hohlzylinder der kurze Hebelabschnitt gegen das freie Ende des ersten Hebels gedrückt wird, wobei der erste Hebel um den ersten Drehpunkt gedreht wird und gegen die verformbare Mantelfläche des Hohlzylinders unter Verformung der Mantelfläche gedrückt wird und die mindestens eine Ampulle im Hohlzylinder gegen das nicht verformbare Stützelement gebrochen wird.

Ein erfindungsgemäßes Verfahren zur Öffnung von Ampullen mit der bespielhaft beschriebenen Vorrichtung kann beispielsweise durch folgende chronologische Schritte gekennzeichnet sein:
a) der lange Hebelabschnitt des zweiten Hebels wird entgegengesetzt zur Aufnahme, die als Hohlzylinder ausgebildet ist, um den zweiten Drehpunkt manuell gedreht,
b) der kurze Hebelabschnitt drückt bei seiner Drehbewegung um den zweiten Drehpunkt auf das freie Ende des ersten Hebels,
c) das freie Ende des ersten Hebels wird auf die verformbare Außenfläche des Hohlzylinders gedrückt,
d) die verformte Fläche des Hohlzylinders wird auf die Mantelfläche der Ampulle gedrückt, wobei die Ampulle gegen das nicht verformbare Stützelement gedrückt wird und zerbricht, wobei die Glassplitter auf das flüssigkeitsdurchlässige Siebelement fallen und
e) die Flüssigkeit fließt aus der gebrochenen Ampulle durch das Siebelement in den Trichter.

Weiterhin ist eine Full-Prepac-Zementiervorrichtung mit der Vorrichtung zum Öffnen von Glasampullen erfindungsgemäß. Die Full-Prepac-Zementiervorrichtung ist zusammengesetzt aus
a) einem Hohlzylinder als Aufnahme mit mindestens einer verformbaren Fläche zur Aufnahme von mindestens einer Glasampulle,
b) mindestens einer Glasampulle, die Monomerflüssigkeit enthält und die in dem Hohlzylinder angeordnet ist,
c) einem nicht verformbaren Stützelement gegenüber der verformbaren Mantelfläche des Hohlzylinders,
d) einer flüssigkeitsdurchlässigen Siebplatte, die unter unterhalb des Hohlzylinders angeordnet ist,
e) einem Trichter der unterhalb der Siebplatte angeordnet ist,
f) einem ersten Hebel, der an einem ersten Gelenk derart drehbar angelenkt ist, dass das freie Ende des ersten Hebels auf der elastisch verformbaren Außenfläche des Hohlzylinders anliegt,
g) einem zweiten Hebel, der durch ein zweites Gelenk in einen kurzen Hebelabschnitt und einem langen Hebelabschnitt geteilt ist, wobei der Drehpunkt des zweiten Hebels höher angeordnet ist als der Drehpunkt des ersten Hebels,
h) der zweite Hebel parallel zum Hohlzylinder angeordnet ist, wobei der kurze Hebelabschnitt parallel zum ersten Hebel angeordnet ist,
i) einer hohlzylinderförmigen Kartusche,
j) Zementpulver, das in dem Innenraum der Kartusche angeordnet ist,
k) einem flüssigkeitsdurchlässigen Verbindungsmittel, das den Trichter mit dem Hohlraum der Kartusche verbindet,
l) einer manuell zu betätigenden Rühreinheit, wobei das Rührelement in der Kartusche angeordnet ist,
m) einem axial in der Kartusche verschiebbaren Kolben, der gasdicht den Innenraum der Kartusche verschließt,
n) einem gasdurchlässigen Verbindungsmittel im Kolben, der den Innenraum der Kartusche mit der äußeren Atmosphäre gasdurchlässig verbindet, und
o) optional einem Fußteil, das mit dem Hohlzylinder, der Kartusche, dem ersten Hebel und dem zweiten Hebel verbunden ist.

Eine zweite alternative Full-Prepac-Zementiervorrichtung mit der Vorrichtung zum Öffnen von Glasampullen kann beispielsweise zusammengesetzt sein aus:
a) einem Hohlzylinder mit mindestens einer verformbaren Fläche zur Aufnahme von mindestens einer Glasampulle,
b) mindestens einer Glasampulle, die Monomerflüssigkeit enthält und die in dem Hohlzylinder angeordnet ist,
c) einem nicht verformbaren Stützelement gegenüber der verformbaren Mantelfläche des Hohlzylinders,
d) einer flüssigkeitsdurchlässigen Siebplatte, die unter unterhalb des Hohlzylinders angeordnet ist,
e) einem Trichter der unterhalb der Siebplatte angeordnet ist,
f) einem ersten Hebel, der an einem ersten Gelenk derart drehbar angelenkt ist, dass das freie Ende des ersten Hebels auf der elastisch verformbaren Außenfläche des Hohlzylinders anliegt,
g) einem zweiten Hebel, der durch ein zweites Gelenk in einen kurzen Hebelabschnitt und einem langen Hebelabschnitt geteilt ist, wobei der Drehpunkt des zweiten Hebels höher angeordnet ist als der Drehpunkt des ersten Hebels,
h) der zweite Hebel parallel zum Hohlzylinder angeordnet ist, wobei der kurze Hebelabschnitt parallel zum ersten Hebel angeordnet ist,
i) einer hohlzylinderförmigen Kartusche,
j) Zementpulver, das im Innenraum der Kartusche angeordnet ist,
k) einem flüssigkeitsdurchlässigen ersten Verbindungsmittel, das den Trichter mit einer manuell oder auch durch Federkraft angetriebenen Kolbenpumpe verbindet,
l) einem flüssigkeitsdurchlässigen zweiten Verbindungsmittel, das die Kolbenpumpe mit dem Innenraum der Kartusche flüssigkeitsdurchlässig verbindet,
m) einer manuell zu betätigenden Rühreinheit, wobei das Rührelement in der Kartusche angeordnet ist,
n) einem axial in der Kartusche verschiebbaren Kolben, der gasdicht den Innenraum er Kartusche verschließt,
o) einem gasdurchlässigen zweiten Verbindungsmittel im Kolben, der den Innenraum der Kartusche mit der äußeren Atmosphäre verbindet, und
p) optional einem Fußteil, das mit dem Hohlzylinder, der Kartusche, dem ersten Hebel und dem zweiten Hebel verbunden ist.

Eine dritte erfindungsgemäße Full-Prepac-Zementiervorrichtung mit der Vorrichtung zum Öffnen von Glasampullen ist beispielhaft zusammengesetzt aus
a) einem Hohlzylinder als Aufnahme mit mindestens einer verformbaren Fläche zur Aufnahme von mindestens einer Glasampulle,
b) mindestens einer Glasampulle, die Monomerflüssigkeit enthält und die in dem Hohlzylinder angeordnet ist,
c) einem nicht verformbaren Stützelement gegenüber der verformbaren Mantelfläche des Hohlzylinders,
d) einer flüssigkeitsdurchlässigen Siebplatte, die unter unterhalb des Hohlzylinders angeordnet ist,
e) einem Trichter der unterhalb der Siebplatte angeordnet ist,
f) einem ersten Hebel, der an einem ersten Gelenk derart drehbar angelenkt ist, dass das freie Ende des ersten Hebels auf der elastisch verformbaren Außenfläche des Hohlzylinders anliegt,
g) einem zweiten Hebel, der durch ein zweites Gelenk in einen kurzen Hebelabschnitt und einem langen Hebelabschnitt geteilt ist, wobei der Drehpunkt des zweiten Hebels höher angeordnet ist als der Drehpunkt des ersten Hebels,
h) der zweite Hebel parallel zum Hohlzylinder angeordnet ist, wobei der kurze Hebelabschnitt parallel zum ersten Hebel angeordnet ist,
i) einer hohlzylinderförmigen Kartusche,
j) Zementpulver, das im Innenraum der Kartusche angeordnet ist,
k) einem flüssigkeitsdurchlässigen ersten Verbindungsmittel, das den Trichter mit dem Innenraum der Kartusche flüssigkeitsdurchlässig verbindet,
l) einer manuell zu betätigenden Rühreinheit, wobei das Rührelement in der Kartusche angeordnet ist,
m) einem axial in der Kartusche verschiebbaren Kolben, der gasdicht den Innenraum der Kartusche verschließt,
n) einem gasdurchlässigen zweiten Verbindungsmittel im Kolben, der den Innenraum der Kartusche mit der äußeren Atmosphäre verbindet,
o) einer manuell oder durch Federkraft angetrieben Vakuumpumpe, die mit einem dritten Verbindungsmittel mit dem gasdurchlässigen zweiten Verbindungsmittel im Kolben verbunden ist, und
p) optional einem Fußteil, das mit dem Hohlzylinder, der Kartusche, dem ersten Hebel und dem zweiten Hebel verbunden ist.

Eine vierte erfindungsgemäße Full-Prepac-Zementiervorrichtung mit der Vorrichtung zum Öffnen von Glasampullen kann beispielsweise zusammengesetzt sein aus
a) einer manuell oder durch Federkraft angetriebenen Saug-Druck-Pumpe, die bei Betätigung synchron pumpt und saugt,
b) einem Hohlzylinder mit mindestens einer verformbaren Fläche zur Aufnahme von mindestens einer Glasampulle,
c) mindestens eine Glasampulle, die Monomerflüssigkeit enthält und die in dem Hohlzylinder angeordnet ist,
d) einem nicht verformbaren Stützelement gegenüber der verformbaren Mantelfläche des Hohlzylinders,
e) einer flüssigkeitsdurchlässigen Siebplatte, die unter unterhalb des Hohlzylinders angeordnet ist,
f) einem Trichter der unterhalb der Siebplatte,
g) einem ersten Hebel, der an einem ersten Gelenk derart drehbar angelenkt ist, dass das freie Ende des ersten Hebels auf der elastisch verformbaren Außenfläche des Hohlzylinders anliegt,
h) einem zweiten Hebel, der durch ein zweites Gelenk in einen kurzen Hebelabschnitt und einem langen Hebelabschnitt geteilt ist, wobei der Drehpunkt des zweiten Hebels höher angeordnet ist als der Drehpunkt des ersten Hebels,
i) der zweite Hebel parallel zum Hohlzylinder angeordnet ist, wobei der kurze Hebelabschnitt parallel zum ersten Hebel angeordnet ist,
j) einer hohlzylinderförmigen Kartusche,
k) Zementpulver, das im Innenraum der Kartusche angeordnet ist,
l) einem flüssigkeitsdurchlässigen ersten Verbindungsmittel, das den Trichter mit einem Druck-Pumpteil der doppelt-wirkenden Pumpe verbindet,
m) einem flüssigkeitsdurchlässigen zweiten Verbindungsmittel, das den Druck-Pumpteil der Saug-Druck-Pumpe mit dem Innenraum der Kartusche verbindet,
n) einer manuell zu betätigenden Rühreinheit, wobei das Rührelement in der Kartusche angeordnet ist,
o) einem axial in der Kartusche verschiebbaren Kolben, der gasdicht den Innenraum der Kartusche verschließt,
p) einem gasdurchlässigen dritten Verbindungsmittel im Kolben, der den Innenraum der Kartusche mit der äußeren Atmosphäre verbindet,
q) einem gasdurchlässigen vierten Verbindungsmittel, das den Saug-Pumpteil der Saug-Druck-Pumpe mit dem gasdurchlässigen dritten Verbindungsmittel im Kolben verbindet, und
r) optional einem Fußteil, das mit dem Hohlzylinder, der Kartusche, dem ersten Hebel und dem zweiten Hebel verbunden ist.

Im Folgenden werden weitere Ausführungsbeispiele der Erfindung anhand von fünf schematisch dargestellten Figuren erläutert, ohne jedoch dabei die Erfindung zu beschränken. Dabei zeigt:
- Figur 1:: eine schematische Seitenansicht einer erfindungsgemäßen Zementiervorrichtung mit einer erfindungsgemäßen Vorrichtung zum Öffnen von Glasampullen;
- Figur 2:: eine schematische Querschnittansicht der Zementiervorrichtung nach Figur 1;
- Figur 3:: eine schematische Detail-Querschnittansicht der erfindungsgemäßen Vorrichtung zum Öffnen von Glasampullen als Teil der Zementiervorrichtung nach den Figuren 1 und 2 vordem Aufbrechen der Glasampulle;
- Figur 4:: eine schematische Detail-Querschnittansicht der erfindungsgemäßen Vorrichtung zum Öffnen von Glasampullen nach Figur 3 nach dem Aufbrechen der Glasampulle; und
- Figur 5:: eine schematische perspektivische Querschnittansicht eines Teils der Zementiervorrichtung nach den Figuren 1 und 2.

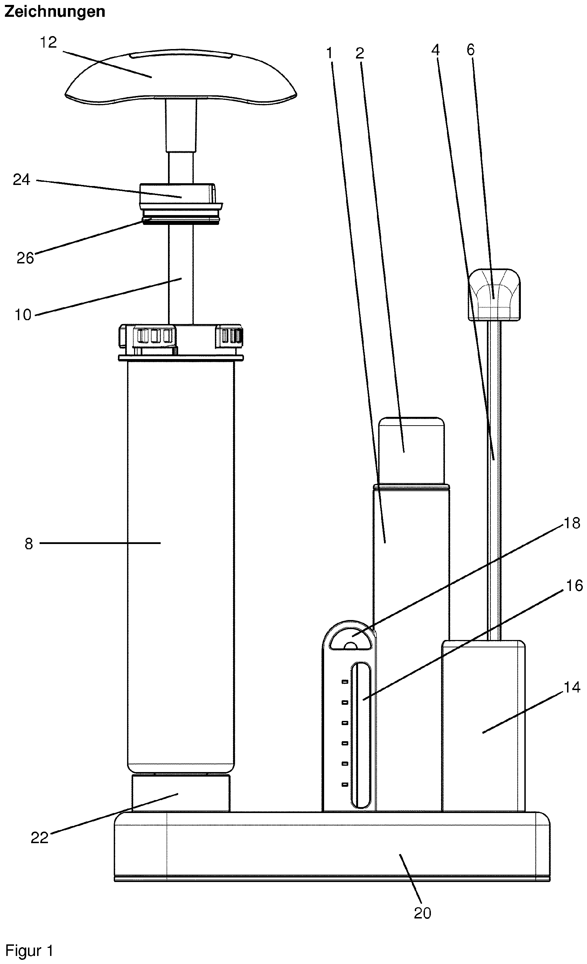

Die Figuren 1 bis 5 zeigen verschiedene Ansichten einer erfindungsgemäßen Zementiervorrichtung und einer erfindungsgemäßen Vorrichtung zum Öffnen von Glasampullen als Teil dieser Zementiervorrichtung. Figur 1 zeigt eine schematische Seitenansicht einer erfindungsgemäßen Zementiervorrichtung mit einer erfindungsgemäßen Vorrichtung zum Öffnen von Glasampullen. Die Zementiervorrichtung weist einen Ampullenbehälter 1 auf, der auf der Oberseite (in Figur 1 oben) mit einem Deckel 2 geschlossen ist. Im Inneren des Ampullenbehälters 1 befindet sich eine Glasampulle (in Figur 1 nicht zu sehen), die innerhalb des Ampullenbehälters 1 beziehungsweise innerhalb der Vorrichtung zum Öffnen der Glasampulle geöffnet werden kann. Der Deckel 2 kann einfach in den Ampullenbehälter 1 eingesteckt werden, um den Innenraum des Ampullenbehälters 1 zu verschließen. Neben dem Ampullenbehälter 1 ist ein Hebel 4 vorgesehen, der in einem Griff 6 endet. Der Hebel 4 kann gegen den Ampullenbehälter 1 mit Hilfe des Griffs 6 manuell in Richtung von dem Ampullenbehälter 1 und dem Deckel 2 weg gedreht beziehungsweise gekippt werden, um die Zementiervorrichtung beziehungsweise die Vorrichtung zum Öffnen der Glasampulle zu bedienen.

Des Weiteren ist neben dem Ampullenbehälter 1 eine Kartusche 8 angeordnet, in der sich ein Knochenzementpulver befindet. Ein Mischrohr 10, das in einem Griff 12 endet, ragt in einen Mischraum der Kartusche 8 hinein, der durch den Innenraum der Kartusche 8 gebildet ist. Das Mischrohr 10 ist dazu drehbar und in Längsrichtung verschiebbar in einer Durchführung gelagert, die ins Innere der Kartusche 8 führt.

Der Hebel 4 ist drehbar gegen den Ampullenbehälter 1 innerhalb eines Gehäuses 14 gelagert. Das Gehäuse 14, die Griffe 6, 12, die Mischstange 10, die Kartusche 8, der Hebel 4, der Deckel 2 und der Ampullenbehälter 1 sind aus Kunststoff gefertigt und können mit einem einfachen Spritzgussverfahren hergestellt werden. Der Ampullenbehälter 1 mündet in eine Verbindungsleitung 16, die den Ampullenbehälter 1 mit dem Mischraum der Kartusche 8 flüssigkeitsdurchlässig verbindet. Die Verbindungsleitung 16 bildet eine Schlaufe 18 beziehungsweise einen Siphon 18, um zu verhindern, dass eine Flüssigkeit aus der geöffneten Glasampulle im Ampullenbehälter 1 direkt nach dem Öffnen der Glasampulle in den Mischraum der Kartusche 8 fließt. Die Verbindungsleitung 16 ist transparent und kann durch ein Fenster im Gehäuse im Bereich der Schlaufe 18 durch den Anwender beobachtet werden. Hierdurch hat der Anwender die Möglichkeit visuell zu prüfen, ob die Flüssigkeit in den Mischraum der Kartusche 8 geleitet wird. Zudem kann eine Skalierung im Bereich des Fensters vorgesehen sein, mit der der Anwender die Menge der eingeführten beziehungsweise vorhandenen Flüssigkeit in dem Ampullenbehälter 1 kontrollieren kann.

Der Ampullenbehälter 1, das Gehäuse 14 und die Kartusche 8 werden von einem Fußteil 20 aus Kunststoff getragen. Das Fußteil 20 kann einteilig mit dem Ampullenbehälter 1 und dem Gehäuse 14 ausgeführt sein. Das Fußteil 20 hat eine ebene Unterseite, so dass die Zementiervorrichtung bequem auf einer ebenen Unterlage, wie einem Tisch, aufgestellt werden kann. In dem Fußteil 20 ist ein Stutzen 22 zur Halterung und Befestigung der Kartusche 8 angeordnet.

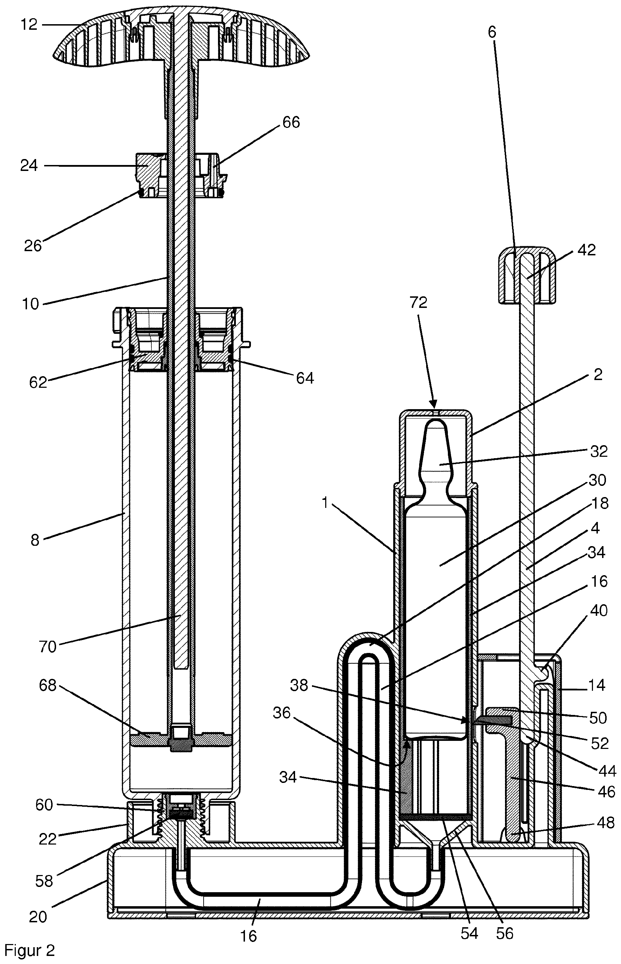

Ein Dichtungskolben 24 als Teil eines zweiteiligen Förderkolbens ist um das Mischrohr 10 herum angeordnet. Das Mischrohr 10 erstreckt sich drehbar und in Längsrichtung verschiebbar durch eine Durchführung in dem Dichtungskolben 24 hindurch. An dem Dichtungskolben 24 ist eine umlaufende Dichtung 26 aus Gummi angeordnet, mit der der Dichtungskolben 24 gegen einen Sterilisationskolben (in Figur 1 nicht zu sehen, in den Figuren 2 bis 5 aber dargestellt) als zweiten Teil des Förderkolbens abgedichtet werden kann, wenn der Dichtungskolben 24 in den Sterilisationskolben eingesteckt wird. Der Sterilisationskolben steckt bereits oben in der Kartusche 8 und schließt den Mischraum der Kartusche 8 nach außen gasdurchlässig aber für das Knochenzementpulver undurchlässig ab. Der Dichtungskolben 24 und der Sterilisationskolben können bis auf die Dichtung 26 und die Dichtungen des Dichtungskolbens als Spritzgussteile aus Kunststoff gefertigt werden.

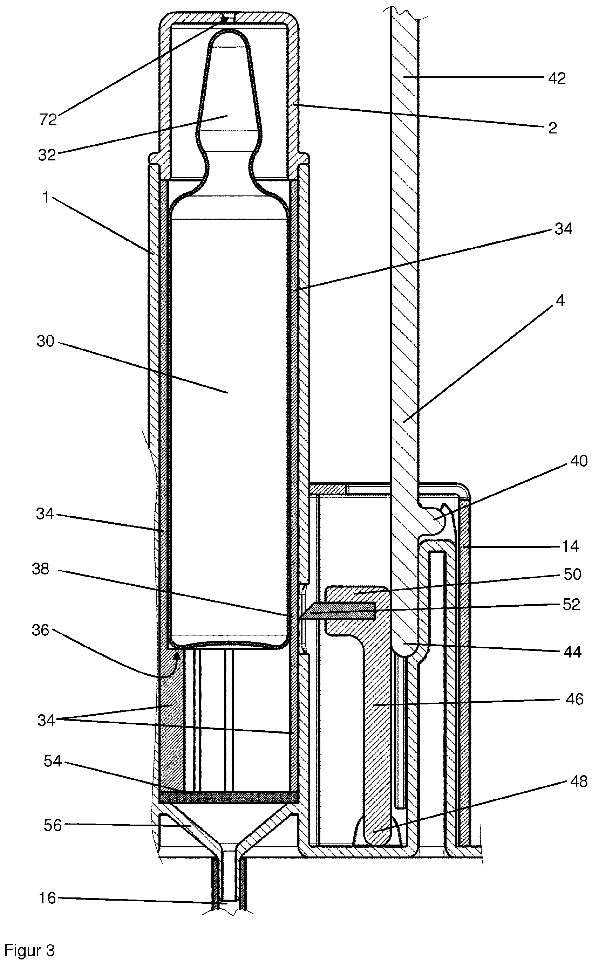

Figur 2 zeigt eine schematische Querschnittansicht der Zementiervorrichtung nach Figur 1. Darin ist zu erkennen, dass sich im Inneren des Ampullenbehälters 1 eine Glasampulle 30 befindet. Die Glasampulle 30 ist mit einer Monomerflüssigkeit gefüllt. Figur 3 zeigt eine schematische Detail-Querschnittansicht der erfindungsgemäßen Vorrichtung zum Öffnen von Glasampullen als Teil der Zementiervorrichtung nach den Figuren 1 und 2 vor dem Aufbrechen der Glasampulle 30. Die Monomerflüssigkeit bildet mit dem Zementpulver aus der Kartusche 8 einen Knochenzementteig, wenn sie miteinander durchmischt werden. Die Glasampulle 30 weist einen Ampullenkopf 32 auf, der üblicherweise zum Öffnen der Glasampulle 30 abgebrochen wird. Da die Glasampulle 30 am Hals dünn ausgeführt ist, führt dies jedoch dazu, dass die Monomerflüssigkeit aus der Glasampulle 30 nur langsam auslaufen kann und daher der Anwender warten muss, bis er die nächsten Schritte zum Bedienen der Zementiervorrichtung durchführen kann.
Die Glasampulle 30 steckt in einem Einsatz 34 aus einem verformbaren Material. Der Einsatz 34 bildet zusammen mit dem Ampullenbehälter 1 eine Aufnahme 1, 34 für die Glasampulle 30. Der Einsatz 34 ist im unteren Bereich mit mehreren Verdickungen ausgeführt, die einen Absatz 36 bilden, auf dem der Ampullenboden aufsitzt. Der Ampullenboden befindet auf der dem Ampullenkopf 32 gegenüberliegenden Seite der Glasampulle 30. Die Glasampulle 30 kann also nur bis zum Absatz 36 in den Einsatz 34 des Ampullenbehälters 1 eingeschoben werden.
Der Ampullenbehälter 1 weist eine seitliche Öffnung auf, in der der Einsatz 34 eine verformbare Seitenwand 38 der Aufnahme bildet. An dieser Stelle kann die Glasampulle 30 geöffnet beziehungsweise aufgebrochen werden, indem ein Druck durch die verformbare Seitenwand 38 auf die Glasampulle 30 knapp oberhalb des Ampullenbodens wirkt. Wenn der Ampullenboden der Glasampulle 30 abgebrochen beziehungsweise die Glasampulle 30 geöffnet wird, kann die Monomerflüssigkeit aus der geöffneten Glasampulle 30 im vollen Querschnitt ausfließen, so dass die Monomerflüssigkeit schnell im vollen Umfang zur Weiterverarbeitung zur innerhalb der Zementiervorrichtung beziehungsweise innerhalb der Vorrichtung zum Öffnen der Glasampulle 30 der Zementiervorrichtung zur Verfügung steht.

Um die verformbare Seitenwand 38 zu verformen und dadurch die Glasampulle 30 aufzubrechen, wird der Hebel 4 verwendet, der über den Griff 6 bedienbar ist und der um eine Achse 40 gedreht werden kann. Der Hebel 4 ist schwenkbar beziehungsweise drehbar um die Achse 40 gegen das Gehäuse 14 gelagert. Die Achse 40 teilt den Hebel 4 in einen langen Hebelarm 42, an dem der Griff 6 befestigt ist und einen kurzen Hebelarm 44 der innerhalb des Gehäuses 14 angeordnet ist. Vorliegend kann der lange Hebelarm 42 nur von dem Ampullenbehälter 1 weg bewegt werden und nicht auf diesen zu, da der kurze Hebelarm 44 an dem Fußteil 20 beziehungsweise dem Gehäuse 14 an der dem Ampullenbehälter 1 gegenüberliegenden Seite (in Figur 2 und 3 rechts) anliegt und so eine Drehung in diese Richtung blockiert.

Der kurze Hebelarm 44 des Hebels 4 liegt auf seiner dem Ampullenbehälter 1 zugewandten Seite an einem weiteren kürzeren Hebel 46 an, der über ein Gelenk 48 beziehungsweise eine Achse 48 drehbar um die Achse 48 mit dem Fußteil 20 der Zementiervorrichtung beziehungsweise der Vorrichtung zum Öffnen der Glasampulle 30 verbunden ist. Dieser weitere Hebel 46 ist innerhalb des Gehäuses 14 angeordnet. Das freie Hebelende 50 des Hebels 46 im Gehäuse 14 kann mit dem kurzen Hebelarm 44 bewegt werden. An der Spitze des freien Hebelendes 50 ist eine Schneidkante 52 befestigt, die an der verformbaren Seitenwand 38 anliegt. Die Achse 48 des Hebels 46 ist dabei so angeordnet, dass sich das freie Hebelende 50 und damit die Schneidkante 52 in Richtung der verformbaren Seitenwand 38 und in Richtung des Fußteils 20 bewegt. Dadurch wird erreicht, dass die Kraft, die von der Schneidkante 52 durch die verformbare Seitenwand 38 auf die Glasampulle 30 ausgeübt werden kann, die Glasampulle 30 auch leicht in Richtung des Absatzes 36 presst und somit die Glasampulle 30 in die Aufnahme 1, 34 hineindrückt.

Unterhalb des Absatzes 36 ist ein Sieb 54 und/oder ein Filter 54 angeordnet, mit dem oder mit denen Glassplitter der geöffneten beziehungsweise aufgebrochenen Glasampulle 30 zurückgehalten werden. Der Abstand zwischen dem Absatz 36 und dem Sieb 54 und/oder Filter 54 ist größer als der Außendurchmesser der Glasampulle 30, so dass der abfallende Ampullenboden sich in dem Zwischenraum drehen kann und den Ausfluss der Monomerflüssigkeit aus der geöffneten Glasampulle 30 nicht behindert. Unterhalb des Siebs 54 und/oder Filters 54 ist ein Trichter 56 angeordnet, der in die Verbindungsleitung 16 mündet. Zusätzlich kann am Eingang von der Aufnahme 1, 34 in die Verbindungsleitung 16 auch noch ein Ventilelement (nicht gezeigt) vorgesehen sein, das mit einem Drehhebel geöffnet beziehungsweise geschlossen werden kann.

Die Verbindungsleitung 16 leitet die Monomerflüssigkeit über die Schlaufe 18 zur Kartusche 8. In der Einmündung der Verbindungsleitung 16 in die Kartusche 8 ist ein pulverundurchlässiger aber für die Monomerflüssigkeit durchlässiger Filter 58 angeordnet. Dieser Filter 58 verhindert, dass Zementpulver aus dem Mischraum der Kartusche 8 in die Verbindungsleitung 16 gelangt, dort mit der Monomerflüssigkeit reagiert und dann in der Verbindungsleitung 16 aushärtet und dadurch die Verbindungsleitung 16 verstopft, noch bevor die Monomerflüssigkeit vollständig oder in der gewünschten Menge in die Kartusche 8 eingeleitet wurde. Der Filter 58 ist in einem Stutzen 60 mit einem Außengewinde vorgesehen. Auf diesen Stutzen 60 ist die Kartusche 8 aufgeschraubt, die dazu ein passendes Innengewinde aufweist.

Am oberen Ende der Kartusche 8 ist innerhalb der Kartusche 8 der Sterilisationskolben 62 angeordnet, der gegen die Innenwände der Kartusche 8 mit zwei umlaufenden Dichtungsringen 64 aus Gummi abgedichtet ist. Der Sterilisationskolben 62 ist gasdurchlässig, so dass durch den Sterilisationskolben 62 das Innere der Kartusche 8, also der Mischraum, mit einem sterilisierenden Gas, wie beispielsweise Ethylenoxid, sterilisiert werden kann. Der Sterilisationskolben 62 begrenzt den Mischraum im Inneren der Kartusche 8 nach oben. Der Sterilisationskolben 62 kann in Längsrichtung innerhalb der Kartusche 8 bewegt werden (in Figur 2 von oben nach unten), um das fertige Zementgemisch aus der Kartusche 8 auszutreiben. In der in Figur 2 gezeigten Stellung ist der Sterilisationskolben 62 jedoch arretiert, um eine ungewollte Bewegung des Sterilisationskolbens 62 zu vermeiden. Der Dichtungskolben 24 und der Sterilisationskolben 62 bilden gemeinsam einen zweiteiligen Förderkolben 24, 62 zum Austreiben des Inhalts aus der Kartusche 8. Der Dichtungskolben 24 kann von oben in den Sterilisationskolben 62 eingesteckt werden, um das Kolbensystem 24, 62, beziehungsweise den Förderkolben 24, 62 abzudichten. In dem Dichtungskolben 24 befindet sich eine Durchführung 66 zum Anschließen einer Vakuumquelle. Mit der Vakuumquelle kann der Mischraum in der Kartusche 8 evakuiert werden und zudem die Monomerflüssigkeit aus dem Ampullenbehälter 1 beziehungsweise der Verbindungsleitung 16 in die Kartusche 8 gesaugt werden.

Im Inneren der Kartusche 8, also im Mischraum, befindet sich eine Mischeinrichtung mit mehreren Mischflügeln 68, die an dem Mischrohr 10 befestigt sind. In dem Mischrohr 10 befindet sich zur Stabilisierung eine Stange 70. Mit dem Mischrohr 10 kann die Mischeinrichtung beziehungsweise können die Mischflügel 68 in dem Mischraum gedreht werden und in Längsrichtung (in Figur 2 von oben nach unten) bewegt werden, um den Inhalt der Kartusche 8 beziehungsweise die Monomerflüssigkeit mit dem Zementpulver zu durchmischen. Damit die Monomerflüssigkeit gut aus dem Ampullenbehälter 1 ausfließen kann, ist in dem Deckel 2 ein Belüftungsdurchgang 72 vorgesehen, durch den Luft in den Ampullenbehälter 1 nachströmen kann, wenn die Monomerflüssigkeit über den Trichter 56 und die Verbindungsleitung 16 in die Kartusche 8 eingeleitet wird. Im Bereich der Innenwand des Einsatzes 34 können hierzu Nuten vorgesehen sein, durch die Luft an der Glasampulle 30 vorbei nachströmen kann.

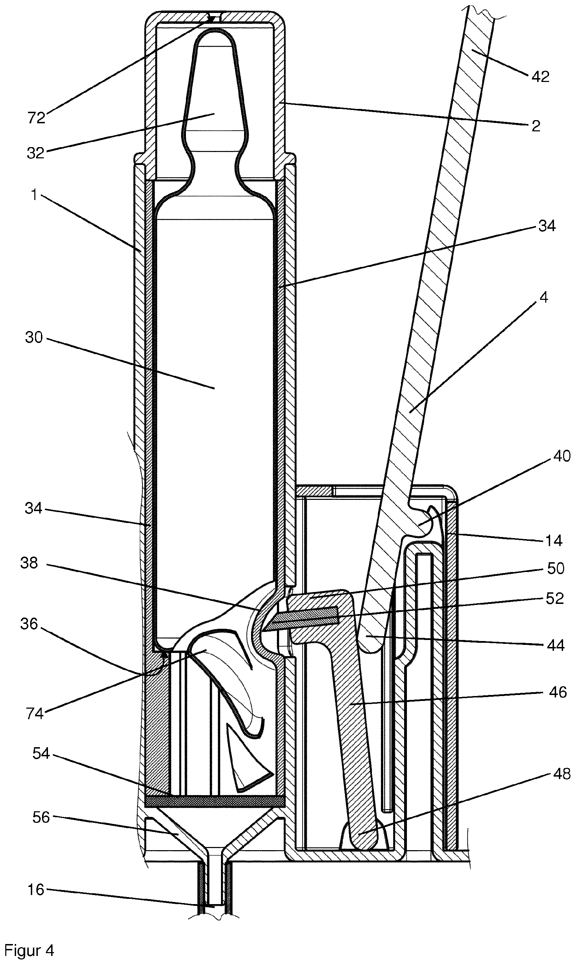

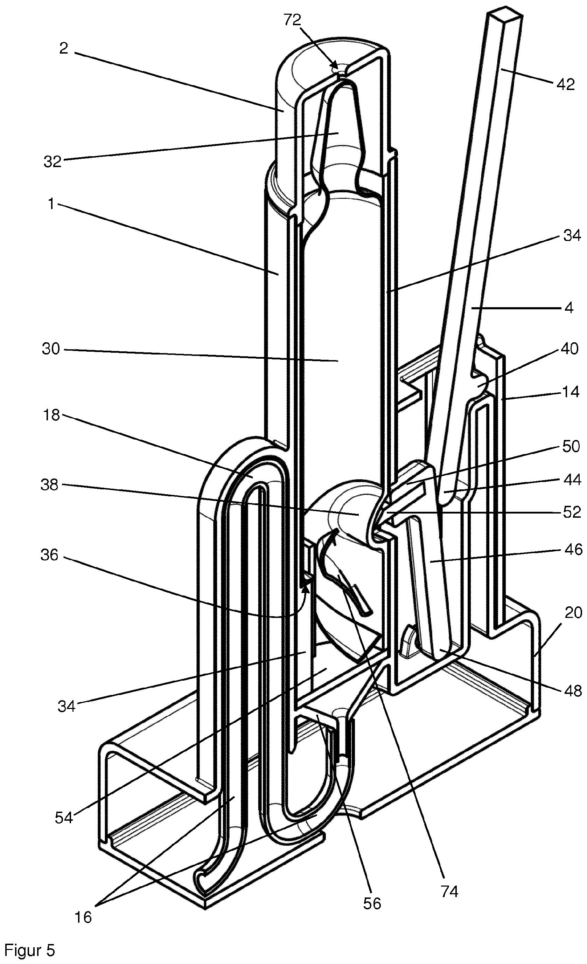

Figur 4 zeigt eine schematische Detail-Querschnittansicht der erfindungsgemäßen Vorrichtung zum Öffnen von Glasampullen nach Figur 3 nach dem Aufbrechen der Glasampulle 30 und Figur 5 eine schematische perspektivische Querschnittansicht eines Teils der Zementiervorrichtung nach den Figuren 1 und 2 mit geöffneter Glasampulle 30. Der Ampullenboden 74 ist von der Glasampulle 30 abgebrochen und damit die Glasampulle 30 in der Zementiervorrichtung beziehungsweise der Vorrichtung zum Öffnen der Glasampulle 30 geöffnet. Dazu wurde der Hebel 4 manuell gekippt, so dass sich der kleinere Hebel 46 im Inneren des Gehäuses 14 mit der Schneidkante 52 auf die verformbare Seitenwand 38 gedrückt hat, bis der Ampullenboden 74 abgebrochen ist.

Damit sich der Ampullenbehälter 1 auf der der verformbaren Seitenwand 38 gegenüberliegenden Seite aufgrund der Krafteinwirkung durch den unteren Hebel 46 nicht zu leicht verformen kann, ist ein Stützelement vorgesehen, das in dem vorliegenden Beispiel durch das Gehäuse gebildet ist, das die Schlaufe 18 der Verbindungsleitung 16 umgibt und das einteilig mit dem Ampullenbehälter 1 ausgeführt ist. Der Ampullenbehälter 1 selbst kann aber auch ohne weiteres das Stützelement bilden. Hierzu muss der Ampullenbehälter 1 lediglich ausreichend stabil sein, also beispielsweise eine ausreichende Wandstärke aufweisen, damit sich die der verformbaren Seitenwand 38 gegenüberliegende Wandung des Ampullenbehälters 1 nicht aufgrund des von dem Hebel 46 ausgeübten und von der Schneidkante 52 und der Glasampulle 30 vermittelten Drucks verformen kann.

Die Monomerflüssigkeit aus der Glasampulle 30 läuft aus und kann zum Mischen mit dem Zementpulver in der Kartusche 8 verwendet werden. Nach dem Aufbrechen der Glasampulle 30 ist die Monomerflüssigkeit in dem Ampullenbehälter 1 verfügbar und kann durch die Verbindungsleitung 16 in den Innenraum der Kartusche 8 geleitet werden, in dem ein Unterdruck im Innenraum der Kartusche 8 verwendet wird, um die Monomerflüssigkeit aus dem Ampullenbehälter 1 in den Innenraum der Kartusche 8 zu saugen. Dieser Unterdruck kann mit einer Pumpe (nicht gezeigt) erzeugt werden. Alternativ kann die Monomerflüssigkeit auch mit einem geeigneten Aufbau (einer Druckpumpe) in die Kartusche 8 hineingedrückt werden. Im Innenraum der Kartusche 8 kann die Monomerflüssigkeit dann mit dem Zementpulver mit den Mischflügeln 68 unter Vakuum beziehungsweise unter Unterdruck gemischt werden, um den Knochenzement beziehungsweise eine Knochenzementpaste zu erzeugen.

Wenn die Ausgangskomponenten im Innenraum der Kartusche 8 mit den Mischflügeln 68 gemischt wurden, wird das Mischrohr 10 soweit es geht aus dem Innenraum der Kartusche 8 nach oben herausgezogen und kann dann an einer Sollbruchstelle abgebrochen werden. Der Dichtungskolben 24 wird gegen den Sterilisationskolben 62 gedreht und somit die Gasdurchführung durch den Dichtungskolben 24 verschlossen. Die Vakuumquelle wird vom Dichtungskolben 24 getrennt. Die Kartusche 8 wird vom Fußteil 20 abgeschraubt und in das Innengewinde der Kartusche 8 ein Austragsrohr (nicht gezeigt) eingeschraubt, durch das der gemischte Knochenzement appliziert werden kann. Der aus dem Sterilisationskolben 62 und dem Dichtungskolben 24 zusammengesetzte Förderkolben 24, 62 wird entrastet und kann mit einem Applikationsgerät (nicht gezeigt) ins Innere der Kartusche 8 getrieben werden. Dadurch wird der Inhalt der Kartusche 8, also der gemischte Knochenzement aus der gegenüberliegenden Öffnung und durch das aufgeschraubte Austragsrohr ausgepresst.

Die Bauteile der Zementiervorrichtung können bis auf die Glasampulle 30, den Filter 58 und die Ausgangskomponenten des Knochenzements durch Spritzgießen aus Kunststoff gefertigt werden. Die Leitungen 16, 18 können aus einem anderen Kunststoff bestehen.

Mit der beschriebenen Zementiervorrichtung können die beiden Ausgangskomponenten des Knochenzements gelagert und zu einem beliebigen späteren Zeitpunkt unter Vakuum gemischt werden. Es ist kein interner Energiespeicher, wie eine Batterie oder eine gespannte Feder, zum Öffnen der Glasampulle 30 notwendig. Die hierzu notwendige Energie wird manuell aufgebracht.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Ampullenbehälter
- 2: Deckel
- 4: Hebel
- 6: Griff
- 8: Kartusche
- 10: Mischrohr
- 12: Griff
- 14: Gehäuse
- 16: Verbindungsleitung
- 18: Schlaufe / Siphon
- 20: Fußteil
- 22: Stutzen
- 24: Dichtungskolben
- 26: Dichtung
- 30: Glasampulle
- 32: Ampullenkopf
- 34: Einsatz
- 36: Absatz
- 38: Verformbare Seitenwand
- 40: Drehachse / Lagerung
- 42: Langer Hebelarm
- 44: Kurzer Hebelarm
- 46: Hebel
- 48: Drehachse / Lagerung
- 50: Freies Hebelende
- 52: Kante
- 54: Sieb / Filter
- 56: Trichter
- 58: Pulverundurchlässiger und Flüssigkeitsdurchlässiger Filter
- 60: Stutzen mit Außengewinde
- 62: Sterilisationskolben
- 64: Dichtung
- 66: Durchführung
- 68: Mischflügel
- 70: Stange
- 72: Belüftungsdurchgang
- 74: Ampullenboden

## Patentansprüche

1. Vorrichtung zum manuellen Öffnen von Glasampullen (30) innerhalb der Vorrichtung, wobei die Vorrichtung die folgenden Teile aufweist:
eine Aufnahme (1, 34) mit zumindest bereichsweise geschlossenen Seitenwänden (34, 38) zur Aufnahme der Glasampulle (30), wobei die Aufnahme (1, 34) zumindest eine verformbare geschlossene Seitenwand (38) aufweist und gegenüber der verformbaren Seitenwand (38) ein Stützelement vorgesehen ist,
ein Sieb (54) und/oder einen Filter (54) das, der oder die unterhalb der Aufnahme (1, 34) angeordnet ist oder sind, so dass der Inhalt der geöffneten Glasampulle (30) durch das Sieb (54) und/oder den Filter (54) fließt, einen ersten Hebel (46), der um eine erste Achse (48) drehbar gegen die Aufnahme (1, 34) gelagert ist, wobei ein freies Ende (50) des ersten Hebels (46) gegen die verformbare Seitenwand (38) der Aufnahme (1, 34) drückbar ist, einen zweiten Hebel (4), der um eine zweite Achse (40) drehbar gegen die Aufnahme (1, 34) gelagert ist, wobei die zweite Achse (40) den zweiten Hebel (4) in einen kurzen Hebelarm (44) und einen langen Hebelarm (42) teilt, wobei ein Ende des kurzen Hebelarms (44) durch manuelles Bedienen des langen Hebelarms (42) gegen den ersten Hebel (46) zu drücken ist, so dass das freie Ende (50) des ersten Hebels (46) gegen die verformbare Seitenwand (38) drückt und diese derart verformt, dass eine in der Aufnahme (1, 34) befindliche und zur Aufnahme (1, 34) passende Glasampulle (30) durch den Druck des freien Endes (50) des ersten Hebels (46) aufzubrechen ist.

2. Vorrichtung nach Anspruch 1, **dadurch gekennzeichnet, dass**
die Aufnahme (1, 34) ein Hohlzylinder ist und/oder die Aufnahme (1, 34) aus einem Elastomer besteht oder einen Einsatz (34) aus einem Elastomer umfasst, wobei vorzugsweise das Elastomer eine Shore-Härte größer 60 aufweist, wobei besonders bevorzugt das Elastomer ein Silikonkautschuk oder ein Ethylen-Propylen-Dien-Kautschuk (EPDM) ist.

3. Vorrichtung nach Anspruch 1 oder 2, **dadurch gekennzeichnet, dass**
in der Aufnahme (1, 34) ein Absatz (36) zur Auflage der Glasampulle (30) angeordnet ist, wobei der Absatz (36) kleiner als die Hälfte der Fläche des Ampullenbodens (74) oder des Ampullenquerschnitts ist.

4. Vorrichtung nach Anspruch 3, **dadurch gekennzeichnet, dass**
der Absatz (36) derart in der Aufnahme (1, 34) angeordnet ist, dass der Abstand zwischen dem Absatz (36) und dem Sieb (54) und/oder Filter (54) gleich oder größer ist als der Außendurchmesser der einzusetzenden Glasampulle (30).

5. Vorrichtung nach einem der vorangehenden Ansprüche, **dadurch gekennzeichnet, dass**
das freie Ende (50) des ersten Hebels (46) bei einer Bedienung des zweiten Hebels (4) derart auf die verformbare Seitenwand (38) drückt, dass der Vektor der Kraft eine Komponente aufweist, die in Richtung des Siebs (54) und/oder Filters (54) gerichtet ist und/oder die eine in die Aufnahme (1, 34) eingesetzte Glasampulle (30) in die Aufnahme (1, 34) hinein drückt, vorzugsweise in Richtung des Absatzes (36) drückt.

6. Vorrichtung nach einem der vorangehenden Ansprüche, **dadurch gekennzeichnet, dass**
am freien Ende (50) des ersten Hebels (46) auf der zur Aufnahme (1, 34) zugewandten Seite eine Schneidkante (52) angeordnet ist.

7. Vorrichtung nach einem der vorangehenden Ansprüche, **dadurch gekennzeichnet, dass**
das Längenverhältnis des langen Hebelarms (42) zum kurzen Hebelarm (44) mindestens 5 zu 1 ist.

8. Vorrichtung nach einem der vorangehenden Ansprüche, **dadurch gekennzeichnet, dass**
in der Aufnahme (1, 34) eine Glasampulle (30) angeordnet ist, die eine Flüssigkeit enthält, vorzugsweise eine Monomerflüssigkeit zur Herstellung eines medizinischen Knochenzements enthält.

9. Vorrichtung nach einem der vorangehenden Ansprüche, **dadurch gekennzeichnet, dass**
der zweite Hebel (4) in der gleichen Ebene zu drehen ist, wie der erste Hebel (46), wobei die Bewegung des zweiten Hebels (4) in die Bewegung des ersten Hebels (46) eingreift.

10. Vorrichtung nach einem der vorangehenden Ansprüche, **dadurch gekennzeichnet, dass**
die zweite Achse (40) des zweiten Hebels (4) oberhalb der ersten Achse (48) des ersten Hebels (46) angeordnet ist, wobei vorzugsweise die erste Achse (48) des ersten Hebels (46) und die zweite Achse (40) des zweiten Hebels (4) parallel zueinander angeordnet sind.

11. Medizinische Zementiervorrichtung, insbesondere Full-Prepacked-Zementiervorrichtung zum Mischen eines PMMA-Knochenzements, aufweisend eine Vorrichtung nach einem der vorangehenden Ansprüche, ferner aufweisend eine Kartusche (8) mit einem Mischraum enthaltend ein Zementpulver, insbesondere ein Knochenzementpulver, wobei der Mischraum mit der Aufnahme (1, 34) unterhalb des Siebs (54) oder des Filters (54) flüssigkeitsdurchlässig verbunden ist.

12. Zementiervorrichtung nach Anspruch 11, **dadurch gekennzeichnet, dass**
in der Aufnahme (1, 34) eine Glasampulle (30) angeordnet ist, in der eine Monomerflüssigkeit enthalten ist,
eine Pumpe, mit der die Monomerflüssigkeit in die Kartusche (8) zu pumpen ist,
eine Verbindungsleitung (16), mit dem die Monomerflüssigkeit aus der Glasampulle (30) in die Kartusche (8) zu leiten ist,
eine manuell zu bedienende Rühreinheit (68), mit der der Inhalt der Kartusche (8) durchmischbar ist,
einen Förderkolben (24, 62), der in Längsrichtung verschiebbar in der Kartusche (8) vorgesehen ist und mit der gemischt Knochenzement aus der Kartusche (8) auszutreiben ist,
einem gasdurchlässigen Verbindungsmittel (66) im Förderkolben (24, 62), das den Innenraum der Kartusche (8) mit der äußeren Umgebung verbindet, und ein Fußteil (20), das mit der Aufnahme (1, 34), der Kartusche (8) und den Hebeln (4, 46) verbunden ist und mit dem die Zementiervorrichtung auf eine ebene Fläche aufstellbar ist.

13. Zementiervorrichtung nach Anspruch 12, **dadurch gekennzeichnet, dass**
die Pumpe eine Saugdruckpumpe ist wobei der Druck-Pumpenteil der Saugdruckpumpe mit der Aufnahme (1, 34) verbunden ist und der Saug-Pumpenteil der Saugdruckpumpe mit dem Mischraum der Kartusche (8) verbunden ist, vorzugsweise über das gasdurchlässige Verbindungsmittel (66) im Förderkolben (24, 62) mit dem Mischraum der Kartusche (8) verbunden ist.

14. Verfahren zum Öffnen einer Glasampulle (30) in einer Vorrichtung nach einem der Ansprüche 1 bis 13, **dadurch gekennzeichnet, dass**
der lange Hebelarm (42) des zweiten Hebels (4) manuell um die zweite Achse (40) gedreht wird, wodurch der kurze Hebelarm (44) des zweiten Hebels (4) auf das freie Ende (50) des ersten Hebels (46) gedrückt wird, so dass sich der erste Hebel (46) um die erste Achse (48) dreht, und das freie Ende (50) des ersten Hebels (46) auf die verformbare Seitenwand (38) der Aufnahme (1, 34) drückt, die verformbare Seitenwand (38) verformt und durch die Verformung der verformbaren Seitenwand (38) eine Glasampulle (30) in der Aufnahme (1, 34) mechanisch aufgebrochen wird.

15. Verfahren zum Mischen einer Monomer-Flüssigkeit mit einem Knochenzementpulver umfassend ein Verfahren nach Anspruch 14, **dadurch gekennzeichnet, dass**
nach dem Aufbrechen der Glasampulle (30) eine Monomer-Flüssigkeit aus der Glasampulle (30) durch das Sieb (54) und/oder den Filter (54) ausläuft und in einen Mischraum der Kartusche (8) geleitet wird, in dem sich ein Knochenzementpulver befindet, wobei anschließend das Knochenzementpulver und die Monomerflüssigkeit in dem Mischraum der Kartusche (8) gemischt werden.

## Claims

1. Device for manual opening of glass ampoules (30) inside the device, whereby the device comprises the following parts:
a receptacle (1, 34) with at least partly closed side walls (34, 38) for accommodation of the glass ampoule (30), whereby the receptacle (1, 34) comprises at least one deformable closed side wall (38) and a supporting element is provided opposite from the deformable side wall (38),
a screen (54) and/or a
filter (54) that is or are arranged below the receptacle (1, 34) such that the content of the opened glass ampoule (30) flows through the screen (54) and/or the filter (54),
a first lever (46) that is supported against the receptacle (1, 34) such that it can rotate about a first axis (48), whereby a free end (50) of the first lever (46) can be pushed against the deformable side wall (38) of the receptacle (1, 34), a second lever (4) that is supported against the receptacle (1, 34) such that it can rotate about a second axis (40), whereby the second axis (40) subdivides the second lever (4) into a short lever arm (44) and a long lever arm (42),
whereby an end of the short lever arm (44) can be pushed against the first lever (46) by manual actuation of the long lever arm (42) such that the free end (50) of the first lever (46) pushes against the deformable side wall (38) and deforms it appropriately such that a glass ampoule (30) that is situated in the receptacle (1, 34) and matches the receptacle (1, 34) can be broken open by means of the pressure of the free end (50) of the first lever (46).

2. Device according to claim 1, **characterised in that**
the receptacle (1, 34) is a hollow cylinder and/or the receptacle (1, 34) consists of an elastomer or comprises an insert (34) made of an elastomer, whereby the elastomer preferably comprises a Shore hardness of more than 60, whereby the elastomer particularly preferably is a silicone rubber or an ethylene-propylene-diene rubber (EPDM).

3. Device according to claim 1 or 2, **characterised in that**
a ledge (6) for placing-on the glass ampoule (30) is arranged in the receptacle (1, 34), whereby the ledge (6) is smaller than half of the surface area of the ampoule floor (74) or of the ampoule cross-section.

4. Device according to claim 3, **characterised in that**
the ledge (36) is arranged appropriately in the receptacle (1, 34) such that the distance between the ledge (36) and the screen (54) and/or filter (54) is equal to or larger than the external diameter of the glass ampoule (30) to be inserted.

5. Device according to any one of the preceding claims, **characterised in that**
the free end (50) of the first lever (46) pushes appropriately onto the deformable sidewall (38) upon actuation of the second lever (4) such that the vector of the force comprises a component that is directed in the direction of the screen (54) and/or filter (54) and/or that pushes a glass ampoule (30) that is inserted into the receptacle (1, 34) into the receptacle (1, 34), preferably pushes in the direction of the ledge (36).

6. Device according to any one of the preceding claims, **characterised in that**
a cutting edge (52) is arranged on the free end (50) of the first lever (46) on the side facing the receptacle (1, 34).

7. Device according to any one of the preceding claims, **characterised in that**
the length ratio of the long lever arm (42) and the short lever arm (44) is at least 5 to 1.

8. Device according to any one of the preceding claims, **characterised in that**
the receptacle (1, 34) has a glass ampoule (30) arranged in it that contains a liquid, preferably contains a monomer liquid for the production of a medical bone cement.

9. Device according to any one of the preceding claims, **characterised in that**
the second lever (4) can be rotated in the same plane as the first lever (46), whereby the motion of the second lever (4) engages the motion of the first lever (46).

10. Device according to any one of the preceding claims, **characterised in that**
the second axis (40) of the second lever (4) is arranged above the first axis (48) of the first lever (46), whereby the first axis (48) of the first lever (46) and the second axis (40) of the second lever (4) are preferably arranged such as to be parallel with respect to each other.

11. Medical cementing device, in particular full-prepacked cementing device for
the mixing of a PMMA bone cement, comprising a device according to any one of the preceding claims, moreover comprising a cartridge (8) with a mixing space containing a cement powder, in particular a bone cement powder, whereby the mixing space is connected to the receptacle (1, 34) below the screen (54) or the filter (54) in liquid permeable manner.

12. Cementing device according to claim 11, **characterised in that**
a glass ampoule (30), in which a monomer liquid is contained, is arranged in the receptacle (1, 34),
a pump, by means of which the monomer liquid can be pumped into the cartridge (8), a connecting line (16), by means of which the monomer liquid can be guided from the glass ampoule (30) into the cartridge (8),
a manually operated steering unit (68) by means of which the content of the cartridge (8) can be mixed,
a feed plunger (24, 62) that is arranged in the cartridge (8) such that it can be shifted in longitudinal direction and by means of which the mixed bone cement can be expelled from the cartridge (8),
a gas-permeable connecting means (66) in the feed plunger (24, 62) connecting the internal space of the cartridge (8) to its outer surroundings, and a foot part (20) that is connected to the receptacle (1, 34), the cartridge (8), and the levers (4, 46) and by means of which the cementing device can be set up on a level surface.

13. Cementing device according to claim 12, **characterised in that**
the pump is a suction pressure pump, whereby the pressure-pump part of the suction pressure pump is connected to the receptacle (1, 34) and the suction-pump part of the suction pressure pump is connected to the mixing space of the cartridge (8), preferably is connected to the mixing space of the cartridge (8) by means of the gas-permeable connecting means (66) in the feed plunger (24, 62).

14. Method for opening a glass ampoule (30) in a device according to any one of the claims 1 to 13, **characterised in that**
the long lever arm (42) of the second lever (4) is rotated manually about the second axis (40), whereby the short lever arm (44) of the second lever (4) is pushed onto the free end (50) of the first lever (46) such that the first lever (46) rotates about the first axis (48) and the free end (50) of the first lever (46) pushes onto the deformable side wall (38) of the receptacle (1, 34), deforms the deformable side wall (38), and a glass ampoule (30) is broken open mechanically in the receptacle (1, 34) by means of the deformation of the deformable side wall (38).

15. Method for the mixing of a monomer liquid with a bone cement powder comprising a method according to claim 14, **characterised in that** after the glass ampoule (30) is broken open, a monomer liquid leaks from the glass ampoule (30) through the screen (54) and/or the filter (54) and is guided into a mixing space of the cartridge (8), in which a bone cement powder is situated, whereby the bone cement powder and the monomer liquid are subsequently being mixed in the mixing space of the cartridge (8).

## Revendications

1. Dispositif d'ouverture manuelle d'ampoules de verre (30) au sein du dispositif, dans lequel le dispositif présente les parties suivantes :
un logement (1, 34) avec des parois latérales (34, 38) fermées au moins par région pour la réception de l'ampoule de verre (30), dans lequel le logement (1, 34) présente au moins une paroi latérale fermée déformable (38) et un élément d'appui est prévu à l'opposé de la paroi latérale déformable (38), un tamis (54) et/ou un filtre (54) qui est ou sont disposé(s) en dessous du logement (1, 34) de sorte que le contenu de l'ampoule de verre ouverte (30) s'écoule à travers le tamis (54) et/ou le filtre (54),
un premier levier (46) qui est logé contre le logement (1, 34) de manière à pouvoir être tourné autour d'un premier axe (48), dans lequel une extrémité libre (50) du premier levier (46) peut être poussée contre la paroi latérale déformable (38) du logement (1, 34),
un second levier (4) qui est logé contre le logement (1, 34) de manière à pouvoir être tourné autour d'un second axe (40), dans lequel le second axe (40) divise le second levier (4) en un bras de levier court (44) et un bras de levier long (42), dans lequel une extrémité du bras de levier court (44) est à pousser contre le premier levier (46) par maniement manuel du bras de levier long (42) de sorte que l'extrémité libre (50) du premier levier (46) pousse contre la paroi latérale déformable (38) et déforme celle-ci de telle sorte qu'une ampoule de verre (30) se trouvant dans le logement (1, 34) et adaptée au logement (1, 34) est à casser par la pression de l'extrémité libre (50) du premier levier (46).

2. Dispositif selon la revendication 1, **caractérisé en ce que**
le logement (1, 34) est un cylindre creux et/ou le logement (1, 34) se compose d'un élastomère ou comprend un insert (34) en un élastomère, dans lequel l'élastomère présente de préférence une dureté Shore supérieure à 60, dans lequel l'élastomère est de manière particulièrement préférée un caoutchouc de silicone ou un caoutchouc d'éthylène-propylène-diène (EPDM).

3. Dispositif selon la revendication 1 ou 2, **caractérisé en ce que**
un gradin (36) est disposé dans le logement (1, 34) pour le support de l'ampoule de verre (30), dans lequel le gradin (36) est plus petit que la moitié de la face du fond d'ampoule (74) ou de la section transversale d'ampoule.

4. Dispositif selon la revendication 3, **caractérisé en ce que**
le gradin (36) est disposé dans le logement (1, 34) de telle sorte que l'écart entre le gradin (36) et le tamis (54) et/ou filtre (54) est supérieur ou égal au diamètre externe de l'ampoule de verre à insérer (30).

5. Dispositif selon une des revendications précédentes, **caractérisé en ce que** l'extrémité libre (50) du premier levier (46) pousse sur la paroi latérale déformable (38) lors d'un maniement du second levier (4) de telle sorte que le vecteur de la force présente une composante qui est orientée en direction du tamis (54) et/ou filtre (54) et/ou qui pousse dans le logement (1, 34) une ampoule de verre (30) insérée dans le logement (1, 34), pousse de préférence en direction du gradin (36).

6. Dispositif selon une des revendications précédentes, **caractérisé en ce que**
une arête de coupe (52) est disposée à l'extrémité libre (50) du premier levier (46) sur le côté tourné vers le logement (1, 34).

7. Dispositif selon une des revendications précédentes, **caractérisé en ce que**
le rapport de longueur du bras de levier long (42) sur le bras de levier court (44) est d'au moins 5 sur 1.

8. Dispositif selon une des revendications précédentes, **caractérisé en ce que**
une ampoule de verre (30) qui contient un fluide, contient de préférence un fluide monomère pour la fabrication d'un ciment osseux médical, est disposée dans le logement (1, 34).

9. Dispositif selon une des revendications précédentes, **caractérisé en ce que**
le second levier (4) est à tourner dans le même plan que le premier levier (46), dans lequel le mouvement du second levier (4) s'engrène dans le mouvement du premier levier (46).

10. Dispositif selon une des revendications précédentes, **caractérisé en ce que**
le second axe (40) du second levier (4) est disposé au-dessus du premier axe (48) du premier levier (46), dans lequel le premier axe (48) du premier levier (46) et le second axe (40) du second levier (4) sont de préférence disposés parallèlement l'un à l'autre.

11. Dispositif de cimentation médical, notamment dispositif de cimentation à pré-emballage complet pour le mélange d'un ciment osseux de PMMA, présentant un dispositif selon une des revendications précédentes, présentant en outre une cartouche (8) avec un espace de mélange contenant une poudre de ciment, notamment une poudre de ciment osseux, dans lequel l'espace de mélange est relié de manière perméable au fluide au logement (1, 34) en dessous du tamis (54) ou du filtre (54).

12. Dispositif de cimentation selon la revendication 11, **caractérisé en ce que**
une ampoule de verre (30) dans laquelle un fluide monomère est contenu est disposée dans le logement (1, 34),
une pompe avec laquelle le fluide monomère est à pomper dans la cartouche (8),
une conduite de connexion (16) avec laquelle le fluide monomère est à conduire de l'ampoule de verre (30) dans la cartouche (8),
un module d'agitation (68) à maniement manuel avec lequel le contenu de la cartouche (8) peut être mélangé,
un piston d'alimentation (24, 62) qui est prévu dans la cartouche (8) de manière à pouvoir être coulissé dans la direction longitudinale et avec lequel du ciment osseux mélangé est à expulser de la cartouche (8),
un moyen de connexion perméable au gaz (66) dans le piston d'alimentation (24, 62) qui relie l'espace interne de la cartouche (8) à l'environnement extérieur, et
un pied (20) qui est relié au logement (1, 34), à la cartouche (8) et aux leviers (4, 46) et avec lequel le dispositif de cimentation peut être placé sur une surface plane.

13. Dispositif de cimentation selon la revendication 12, **caractérisé en ce que**
la pompe est une pompe aspirante et foulante, dans laquelle la partie de pompe foulante de la pompe aspirante et foulante est reliée au logement (1, 34) et la partie de pompe aspirante de la pompe aspirante et foulante est reliée à l'espace de mélange de la cartouche (8), est de préférence reliée à l'espace de mélange de la cartouche (8) par le biais du moyen de connexion perméable au gaz (66) dans le piston d'alimentation (24, 62).

14. Procédé d'ouverture d'une ampoule de verre (30) dans un dispositif selon une des revendications 1 à 13, **caractérisé en ce que**
le bras de levier long (42) du second levier (4) est tourné manuellement autour du second axe (40), moyennant quoi le bras de levier court (44) du second levier (4) est poussé sur l'extrémité libre (50) du premier levier (46) de sorte que le premier levier (46) tourne autour du premier axe (48), et l'extrémité libre (50) du premier levier (46) pousse sur la paroi latérale déformable (38) du logement (1, 34), déforme la paroi latérale déformable (38) et une ampoule de verre (30) dans le logement (1, 34) est mécaniquement cassée par la déformation de la paroi latérale déformable (38).

15. Procédé de mélange d'un fluide monomère avec une poudre de ciment osseux comprenant un procédé selon la revendication 14, **caractérisé en ce que**
après le cassage de l'ampoule de verre (30), un fluide monomère s'écoule de l'ampoule de verre (30) à travers le tamis (54) et/ou le filtre (54) et est conduit dans un espace de mélange de la cartouche (8) dans lequel une poudre de ciment osseux se trouve, dans lequel la poudre de ciment osseux et le fluide monomère sont ensuite mélangés dans l'espace de mélange de la cartouche (8).
